# EUROPEAN PATENT APPLICATION

(11) **EP 1 424 342 A1**
(43) Date of publication of application: **02.06.2004**
(21) Application number: 02026461.0
(22) Date of filing: 27.11.2002
(51) Int. Cl.: C07K 14/415, A23J 1/12, C12N 15/29, C12N 15/63, C12N 5/10, A61K 47/42, A61K 38/16, A23J 1/00, A23L 1/305, A21D 2/26

(54) **Nucleic acid comprising a sequence encoding a modified glutenin polypeptide**

(71) Applicant: BakeMark Deutschland GmbH, 28215 Bremen (DE); Monsanto Agrar Deutschland GmbH, 40470 Düsseldorf (DE); Unifern GmbH & Co. KG, 59368 Werne (DE); PURATOS N.V., 1702 Groot-Bijgaarden (BE)
(72) Inventor: Hinzmann, Erika, Dr., 10439 Berlin (DE); Wieser, Herbert, Dr., 85356 Freising (DE); Stahl, Ulf, Prof. Dr. Dipl.-Ing., 12161 Berlin (DE)
(74) Representative: UEXKÜLL & STOLBERG

(57) **Abstract**

The present invention is directed to a nucleic acid comprising a sequence encoding a modified glutenin polypeptide, wherein the sequences encoding one or more cysteine residues responsible for intermolecular crosslinking via disulfide bridges were deleted or substituted by sequences encoding other amino acids without amending the reading frame of the coding sequence.

SEQUENCE LISTING

<110> BakeMark Deutschland GmbH
 Monsanto Agrar Deutschlang GmbH
 Uniferm GmbH & Co. KG
 Puratos N.V.

<120> Nucleic Acid comprising a Sequence encoding a modified Glutenin
Polypeptide

<130> P60565

<160> 10

<170> PatentIn version 3.1

<210> 1
<211> 911
<212> DNA
<213> Wheat cultivar Cheyenne

<221> LMW6

<400> 1

gaattcatca tgaagacctt cctcgtcttt gcccttctag ccgttgtggc gacatctgcc 60

attgcacaga tggagactag ctgcatccct ggtttggaga gaccatggca gcaacaacca 120

ttacaacaaa aagagacatt tccacaacaa ccaccatctt cacaacaaca acaaccattt 180

cctcaacaac caccattttt gcagcaacaa ccttcatttt cgcagcaacc actattttca 240

cagaaacaac aaccagttct accacaacaa ccagcatttt cgcagcaaca acaaacagtt 300

ctaccacaac aaccagcatt ttcgcagcaa caacaccaac agcttctgca acaacaaatc 360

cctattgttc atccatccat tttgcagcag ctaaacccgt gcaaggtatt cctccagcag 420

cagtgtagcc ctgtggcaat gccacaacat cttgctaggt cgcagatgtg gcagcagagc 480

agttgcaatg tgatgcagca acaatgttgc caacaattgc cacgaatccc cgaacaatcc 540

cgctatgagg caatccgtgc tatcatcttc tccatcatcc tacaagaaca acaacagggt 600

tttgtccaac ctcagcagca acaaccccaa cagtcggttc aaggtgtcta ccaaccccaa 660

cagcagtcgc agcagcagct cggacaatgt tttttccaac aacctcaaca acaattgggt 720

caacagcctc aacaacaaca ggtacaaaag ggtacctttt tgcagccaca ccagatagct 780

cgccttgagg tgatgacttc cattgcactc cgtaccctgc caacgatgtg cagtgtcaat 840

gtgccgttgt acagctccat cactagtgcg ccattaggcg ttggcagccg agttggtgcc 900

tactgatcga t 911

<210> 2
<211> 298
<212> PRT
<213> Wheat cultivar Cheyenne

<221>  LMW6-Pro

<400>  2

Met Lys Thr Phe Leu Val Phe Ala Leu Leu Ala Val Val Ala Thr Ser
1               5                   10                  15

Ala Ile Ala Gln Met Glu Thr Ser Cys Ile Pro Gly Leu Glu Arg Pro
            20                  25                  30

Trp Gln Gln Gln Pro Leu Gln Gln Lys Glu Thr Phe Pro Gln Gln Pro
        35                  40                  45

Pro Ser Ser Gln Gln Gln Gln Pro Phe Pro Gln Gln Pro Pro Phe Leu
    50                  55                  60

Gln Gln Gln Pro Ser Phe Ser Gln Gln Pro Leu Phe Ser Gln Lys Gln
65                  70                  75                  80

Gln Pro Val Leu Pro Gln Gln Pro Ala Phe Ser Gln Gln Gln Gln Thr
                85                  90                  95

Val Leu Pro Gln Gln Pro Ala Phe Ser Gln Gln Gln His Gln Gln Leu
            100                 105                 110

Leu Gln Gln Gln Ile Pro Ile Val His Pro Ser Ile Leu Gln Gln Leu
        115                 120                 125

Asn Pro Cys Lys Val Phe Leu Gln Gln Gln Cys Ser Pro Val Ala Met
    130                 135                 140

Pro Gln His Leu Ala Arg Ser Gln Met Trp Gln Gln Ser Ser Cys Asn
145                 150                 155                 160

Val Met Gln Gln Gln Cys Cys Gln Gln Leu Pro Arg Ile Pro Glu Gln
                165                 170                 175

Ser Arg Tyr Glu Ala Ile Arg Ala Ile Ile Phe Ser Ile Ile Leu Gln
            180                 185                 190

Glu Gln Gln Gln Gly Phe Val Gln Pro Gln Gln Gln Gln Pro Gln Gln
        195                 200                 205

Ser Val Gln Gly Val Tyr Gln Pro Gln Gln Gln Ser Gln Gln Gln Leu
    210                 215                 220

Gly Gln Cys Phe Phe Gln Gln Pro Gln Gln Gln Leu Gly Gln Gln Pro
225                 230                 235                 240

Gln Gln Gln Gln Val Gln Lys Gly Thr Phe Leu Gln Pro His Gln Ile
                245                 250                 255

Ala Arg Leu Glu Val Met Thr Ser Ile Ala Leu Arg Thr Leu Pro Thr
            260                 265                 270

Met Cys Ser Val Asn Val Pro Leu Tyr Ser Ser Ile Thr Ser Ala Pro
        275                 280                 285

Leu Gly Val Gly Ser Arg Val Gly Ala Tyr
    290                 295

<210>  3
<211>  27
<212>  DNA
<213>  Artificial sequence

<220>
<221>  LMW-E1
<223>  Oligonucleotide

<400>  3

ccggaattca tcatgaagac cttcctc                                          27

<210>  4
<211>  26
<212>  DNA
<213>  Artificial Sequence

<220>
<221>  LMW-C2
<223>  Oligonucleotide

<400>  4

ccatcgatca gtaggcacca actcgg                                           26

<210>  5
<211>  21
<212>  DNA
<213>  Artificial Sequence

<220>
<221>  LMW-Af
<223>  Oligonucleotide

<400>  5

agactagcat ccctggtttg g                                                21

<210>  6
<211>  21
<212>  DNA
<213>  Artificial Sequence

<220>
<221>  LMW-Ar
<223>  Oligonucleotide

<400>  6

ccagggatgc tagtctccat c                                              21


<210>  7
<211>  20
<212>  DNA
<213>  Artificial Sequence

<220>
<221>  LMW-Bf
<223>  Oligonucleotide

<400>  7

gctcggacaa ggttctttcc                                                20


<210>  8
<211>  20
<212>  DNA
<213>  Artificial Sequence

<220>
<221>  LMW-Br
<223>  Oligonucleotide

<400>  8

ggaaagaacc ttgtccgagc                                                20


<210>  9
<211>  908
<212>  DNA
<213>  Wheat cultivar Cheyenne

<221>  LMWa3Δcys2

<400>  9

gaattcatca tgaagacctt cctcgtcttt gcccttctag ccgttgtggc gacatctgcc      60

attgcacaga tggagactag catccctggt ttggagagac catggcagca acaaccatta     120

caacaaaaag agacatttcc acaacaaccg ccatcttcac aacaacaaca accatttcct     180

caacaaccac catttttgca gcaacaacct tcattttcgc agcaaccact attttcacag     240

aaacaacaac cagttctacc acaacaacca gcattttcgc agcaacaaca aacagttcta     300

ccacaacaac cagcattttc gcagcaacaa caccaacagc ttctgcaaca acaaatccct     360

attgttcatc catccatttt gcagcagcta aacccgtgca aggtattcct ccagcagcag     420

tgtagccctg tggcaatgcc acaacatctt gctaggtcgc agatgtggca gcagagcagt     480

tgcaatgtga tgcagcaaca atgttgccaa caattgccac gaatccccga acaatcccgc     540

tatgaggcaa tccgtgctat catcttctcc atcatcctac aagaacaaca acagggtttt 600

gtccaacctc agcagcaaca accccaacag tcggttcaag gtgtctacca accccaacag 660

cagtcgcagc agcagctcgg acaaggttct ttccaacaac ctcaacaaca actgggtcaa 720

caacctcaac aacaacaggt acaaaagggt acctttttgc agccacacca gatagctcgc 780

cttgaggtga tgacttccat tgcactccgt accctgccaa cgatgtgcag tgtcaatgtg 840

ccgttgtaca gctccatcac tagtgcgcca ttaggcgttg gcagccgagt tggtgcctac 900

tgatcgat 908

<210> 10
<211> 297
<212> PRT
<213> Wheat cultivar Cheyenne

<221> LMWa3Δcys2-Pro

<400> 10

Met Lys Thr Phe Leu Val Phe Ala Leu Leu Ala Val Val Ala Thr Ser
1               5                   10                  15

Ala Ile Ala Gln Met Glu Thr Ser Ile Pro Gly Leu Glu Arg Pro Trp
            20                  25                  30

Gln Gln Gln Pro Leu Gln Gln Lys Glu Thr Phe Pro Gln Gln Pro Pro
        35                  40                  45

Ser Ser Gln Gln Gln Gln Pro Phe Pro Gln Gln Pro Pro Phe Leu Gln
    50                  55                  60

Gln Gln Pro Ser Phe Ser Gln Gln Pro Leu Phe Ser Gln Lys Gln Gln
65                  70                  75                  80

Pro Val Leu Pro Gln Gln Pro Ala Phe Ser Gln Gln Gln Gln Thr Val
                85                  90                  95

Leu Pro Gln Gln Pro Ala Phe Ser Gln Gln Gln His Gln Gln Leu Leu
            100                 105                 110

Gln Gln Gln Ile Pro Ile Val His Pro Ser Ile Leu Gln Gln Leu Asn
        115                 120                 125

Pro Cys Lys Val Phe Leu Gln Gln Gln Cys Ser Pro Val Ala Met Pro
    130                 135                 140

Gln His Leu Ala Arg Ser Gln Met Trp Gln Gln Ser Ser Cys Asn Val
145                 150                 155                 160

Met Gln Gln Gln Cys Cys Gln Gln Leu Pro Arg Ile Pro Glu Gln Ser
                165                 170                 175

Arg Tyr Glu Ala Ile Arg Ala Ile Ile Phe Ser Ile Ile Leu Gln Glu
            180                 185                 190

Gln Gln Gln Gly Phe Val Gln Pro Gln Gln Gln Gln Pro Gln Gln Ser
        195                 200                 205

Val Gln Gly Val Tyr Gln Pro Gln Gln Gln Ser Gln Gln Gln Leu Gly
    210                 215                 220

Gln Gly Ser Phe Gln Gln Pro Gln Gln Gln Leu Gly Gln Gln Pro Gln
225                 230                 235                 240

Gln Gln Gln Val Gln Lys Gly Thr Phe Leu Gln Pro His Gln Ile Ala
                245                 250                 255

Arg Leu Glu Val Met Thr Ser Ile Ala Leu Arg Thr Leu Pro Thr Met
            260                 265                 270

Cys Ser Val Asn Val Pro Leu Tyr Ser Ser Ile Thr Ser Ala Pro Leu
        275                 280                 285

Gly Val Gly Ser Arg Val Gly Ala Tyr
    290                 295

## Description

The present invention is directed to a nucleic acid comprising a sequence encoding a modified glutenin polypeptide, wherein the sequences encoding one or more cysteine residues responsible for intermolecular crosslinking via disulfide bridges were deleted or substituted by sequences encoding other amino acids without amending the reading frame of the coding sequence. The present invention further relates to polypeptides encoded by these nucleic acid sequences as well as the use thereof as a gliadin substitute, specifically for the preparation of gliadin-free foodstuff. Finally, the present invention also relates to the use of respective gliadin-free foodstuff for feeding patients suffering from coeliac disease and less severe forms of gluten intolerance.

Coeliac disease is a condition in which the lining of the small intestine is damaged by gluten, a storage protein found in the starchy endosperm of wheat and rye grains as well as in closely related species. The gluten matrix consists of approximately equal mixtures of gliadin and glutenin proteins. Coeliac disease is primarily caused by the gliadin proteins. Specifically, in this disease the villi of the small intestine are destroyed and the lining becomes flattened, seriously impairing nutrient absorption. Typical symptoms are weight loss, foul-smelling diarr-hoe, vomiting, abdominal pain and swelling of the legs. The only cure currently available is a life-long gluten-free diet strictly avoiding all food and pharmaceutical compositions containing wheat, rye and barley.

Further, a number of humans suffer from general intolerance to glutens. The range of intolerance varies greatly although there are no clear clinical symptoms as in Coeliac disease. Many humans therefore benefit from reducing the uptake of gluten.

When wheat flour is mixed with water the gluten proteins develop a unique, visco-elastic structure which enables the preparation of a large number of food products. Flour mixed with a small amount of water develops a dough, from which leavened bread, unleavened bread (for example nan, chapattis), biscuits, cookies, and cakes are made in various parts of the world. When a larger amount of water is added to flour, batters are formed and these are used to make wafers, pancakes and other products. Doughs made from durum wheat are used to make cous cous and pasta products: pizzas, spaghetti, macaroni and the like. In all of these products the gluten matrix traps gas formed during cooking, which allows the developing product to rise/extend through viscous flow and yet maintains the structure (of the product) by developing elastic restraint.

The gliadin proteins comprise a complex mixture of monomeric polypeptides ranging from about 30,000 to 70,000 in molecular weight. The gliadins are often classified into distinct groups, the α/β-, γ- and ω-gliadins.

In contrast, the glutenin proteins have a molecular weight of up to several millions and are among the largest natural polymers known today. They are formed from polypeptide subunits linked via disulphide bonds. The majority of the monomers have a molecular weight which is similar to the gliadins and are called low-molecular-weight (LMW) subunits. The remainder are larger and are referred to as high-molecular-weight (HMW) glutenin subunits. Gliadins and glutenins are conveniently separated by extracting flour with aqueous alcohols, as the gliadins are soluble and the glutenins insoluble under these conditions.

The gliadins impart viscous flow or extensibility to a dough whereas glutenins impart both elasticity and extensibility. The large glutenin molecules contribute more to elasticity and less to extensibility, whereas the smaller glutenin molecules have the reverse effect. Thus the mean molecular weight distribution of the glutenin monomers as well as the ratio of glutenin to gliadin governs the visco-elasticity of the dough. The different foodstuffs of wheat described above require different visco-elastic properties. For example bread requires a balance of extensibility (to allow the dough to rise) and elasticity (to hold the dough volume before it sets in the oven) whereas biscuits require maximum extension (to enable thin dough sheets to be produced) and minimal elasticity (which would cause biscuit shrinkage prior to baking).

The gluten proteins of wheat are also consumed in a number of other products. Isolated gliadins have film-forming properties and are used as a surface layer or coating on confectionery to inhibit stickiness. Gliadins are further applied as a surface coating to pharmaceutical tablets for the same reason, or to inhibit the taste of unpleasant flavours during consumption of the tablet. Individuals with severe forms of Coeliac disease will already be at risk from consumption of these products.

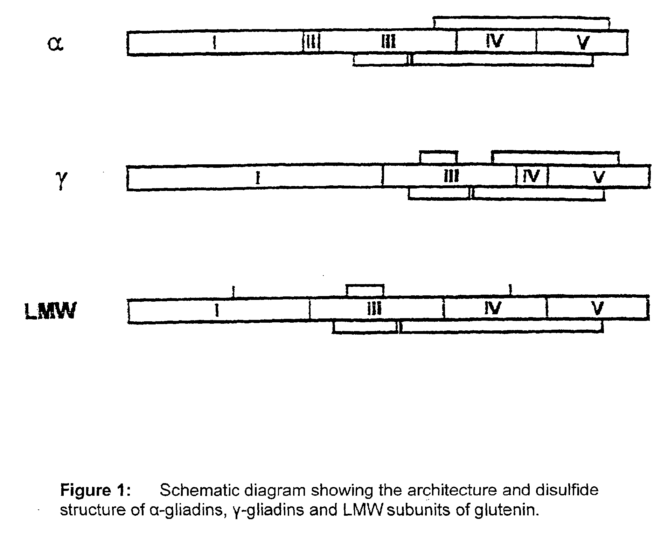

The structures of α/β-gliadins, γ-gliadins and LMW glutenin subunits are closely related and amino acid sequences are homologous in two of five sequence domains (see Figure 1).

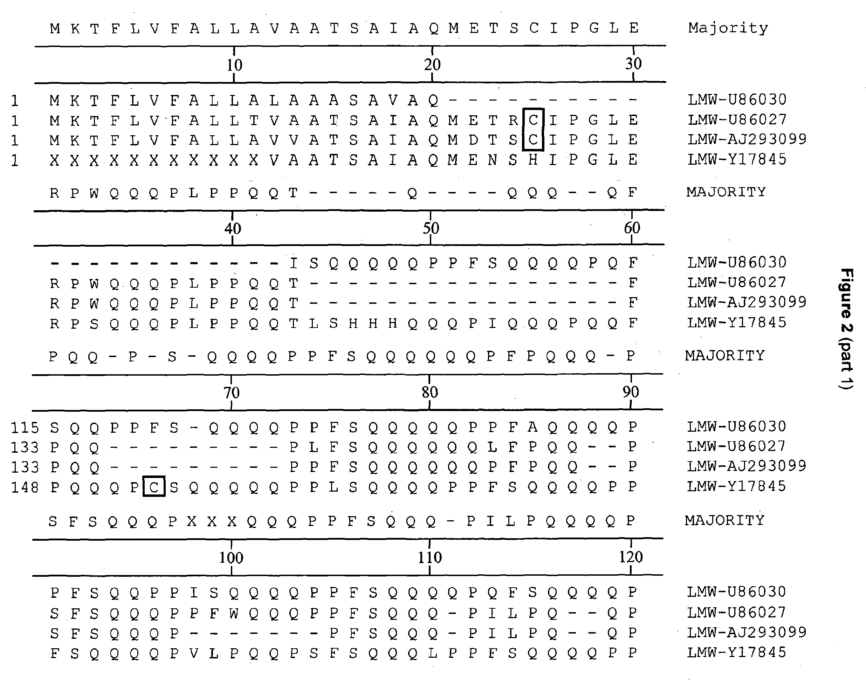

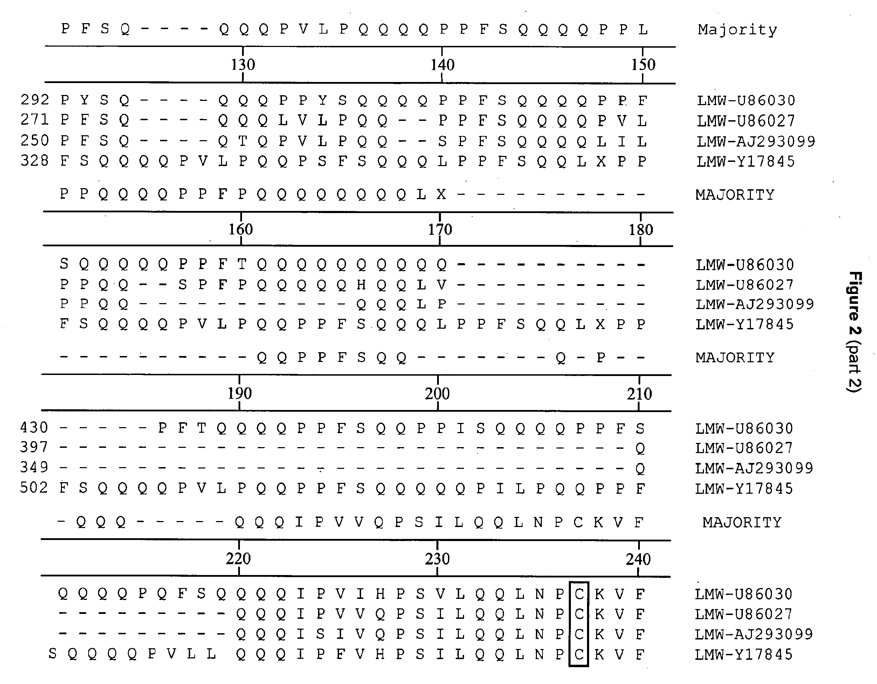

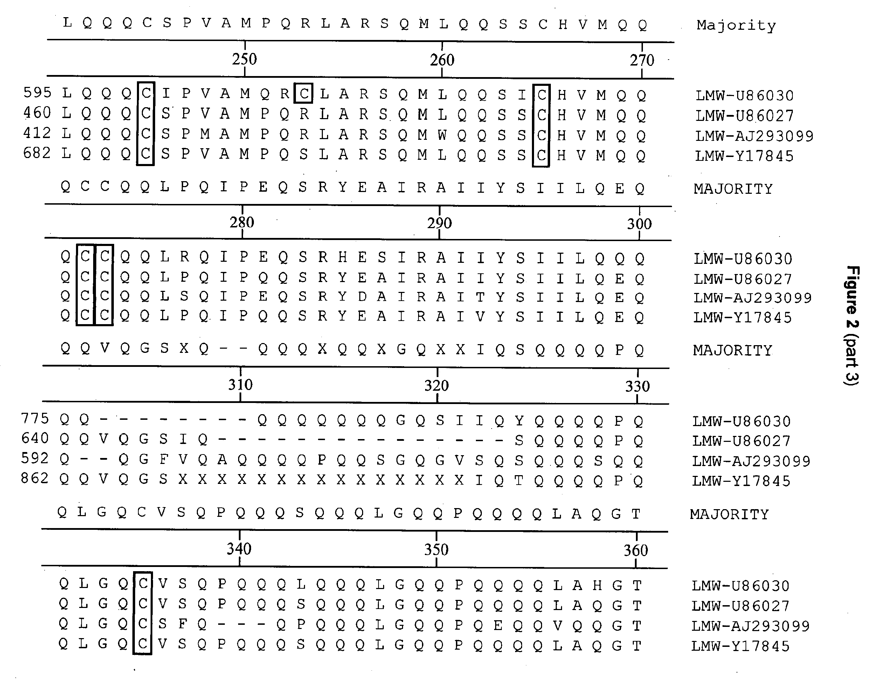

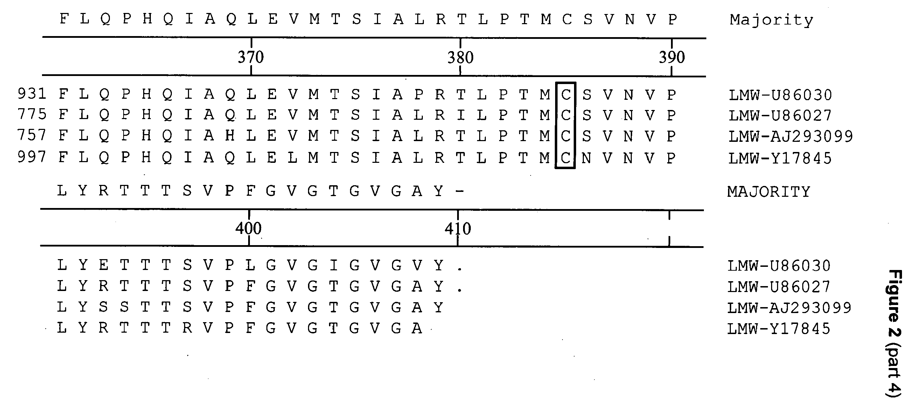

α- and γ-gliadins and LMW glutenins contain 6 or 8 cysteine residues that form 3 or 4 homologous intramolecular disulfide bonds. The location of these cysteine residues in the amino acid sequence is conserved among most LMW glutenin proteins (Figure 2). Two of the cysteine residues present in LMW glutenins form intermolecular disulfide bonds with the cysteine residues of other glutenin proteins. These 2 cysteine residues are thus responsible for the aggregative nature of LMW glutenin subunits. The structure of the storage proteins and the relevance of the cysteine residues for intermolecular crosslinking of glutenins has been analyzed in great detail in the art (Weegels and Hamer, J. Cereal Science, vol. 25 (1997), 155-163; Orsy et al., J. Biological Chemistry, vol. 276 (2001), 32322-32329; Shewry and Tatham, J. Cereal Science, vol. 25 (1997), 207-227; Lindsay et al., J. Cereal Science, vol. 31 (2000), 321-333; and Müller et al., J. Cereal Science, vol. 27 (1998), 109-116).

The use of genetic engineering methods for modifying or improving wheat gluten and wheat products has been suggested in the art. Vasil and Andersen for example suggested to modify wheat gluten by increasing the numbers of HMW-GS genes, to alter the number and position of cysteine residues, to change the physical characteristics of the gluten matrix, to change the composition and arrangement of the repeat motifs in the repetitive domain, to increase the proportion of glutenins in the large polymer fraction, to modify the polymer network, to introduce LMW-GSs with only one available cysteine for intermolecular bonds as polymer chain terminators and to modify the LMW-GSs to achieve modification of wheat quality (Trends Plant Sci., vol. 2 (1997), 292-297).

Modified glutenin genes, wherein sequences encoding a domain which confers the ability to incorporate into a gluten or bind a ligand or other macromolecule were introduced into the gene sequence, have further been reported in the art (WO00/02914).

However, so far no suggestions have been made for improving the gluten composition in such a manner that foodstuffs prepared thereof no longer pose a danger to patients with coeliac disease and other forms of gluten intolerance. The present invention thus relates to the problem of providing a gliadin-reduced or gliadin-free foodstuff having the advantagous characteristics of foodstuff containing wheat storage proteins.

This problem is solved according to the present invention by a nucleic acid comprising a sequence encoding a modified glutenin polypeptide, wherein the sequences encoding one or more cysteine residues responsible for intermolecular crosslinking via disulfide bridges were deleted or substituted by sequences encoding other amino acids without amending the reading frame of the coding sequence.

In further aspects the present invention relates to vectors and host cells comprising respective nucleic acids, the polypeptides encoded thereby as well as the use of the polypeptides for the preparation of a dough, specifically a gliadin-reduced or gliadin-free dough.

The present inventors have surprisingly found that a glutenin polypeptide lacking the cysteine residues responsible for intermolecular crosslinking of glutenins can advantageously be used as a gliadin substitute for example for the preparation of a dough containing a significantly reduced concentration of gliadins or no gliadins at all. The latter foodstuff has the advantagous properties of respective foodstuffs prepared from wheat and can be consumed by patients with coeliac disease without side effects. The present invention thus addresses a long felt need, specifically in the treatment of patients with coeliac disease or general intolerance to gluten.

In accordance with the present invention the term "a nucleic acid encoding a modified glutenin polypeptide, wherein one or more cysteine residues responsible for intermolecular crosslinking via disulfide bridges were deleted or substituted" is used to refer to a nucleic acid encoding a glutenin polypeptide containing a reduced number of cysteine residues when compared to the natural sequence of the respective glutenin polypeptide. Specifically, at least one, preferably all of the sequences encoding cysteine residues responsible for intermolecular crosslinking disulfide bridges are deleted or substituted by nucleotide sequences encoding other amino acids.

In the present application a cysteine residue is referred to as "a cysteine residue responsible for intermolecular crosslinking via disulfide bridges" if the polypeptide containing a respective cysteine residue will form crosslinked di-, oligo- or polymers with other glutenin polypeptides containing respective cysteine residues at appropriate conditions which will allow formation of disulfide bonds, such as under the conditions obtained by expression in yeast as outlined in the examples. Since the domain structure and the crosslinking activity of the various cysteine residues in glutenin genes are well known in the art, one of ordinary skill in the art will have no problem whatsoever to identify the cysteine residues which have to be modified or deleted in any known or newly isolated glutenin gene.

Modified glutenin polypeptides according to the present invention are thus further characterized by the fact that the polypeptides will be maintained as monomers with a size of less than 200 kDa, preferably with a size of less than 100 kDa at conditions which will allow formation of disulfide bonds. At respective conditions the unmodified glutenin polypeptides will thus form oligo- or polymers having a size of up to several million daltons.

Modification of the nucleotide sequence to delete or substitute those sequences encoding a cysteine residue responsible for intermolecular crosslinking via disulfide bridges can be performed according to methods well known in the art, such as site directed mutagenisis of the gene. The modification is preferably performed in such manner that the remaining structure of the glutenin gene is not affected by the deletion of the cysteine residues.

The nucleic acids of the present invention preferably are of DNA or RNA nature.

According to one aspect of the present invention, the nucleic acid encodes a glutenin, preferably a low molecular weight (LMW) glutenin. Numerous wild type genes encoding LMW glutenins are known in the art.

The glutenin genes of the present invention differ from known sulfur rich prolamins, such as wild-type gliadin genes. Comparing the glutenin genes of the present invention with a natural gliadin gene for example shows low (below 50 %, preferably below 30 %) or no homology of the genes in the N-terminal nucleotide sequence of the gene, wherein the N-terminal nucleotide sequence covers at least 15, preferably at least 30 of the nucleotides found at the N-terminus of the coding region.

In a preferred embodiment of the present invention, the nucleic acid comprises a sequence having a homology of at least 75 % to the sequence shown in SEQ ID NO: 9. Preferably the sequence homology is at least 85 % or at least 95 % to the sequence shown in SEQ ID NO: 9. Methods for determining sequence homology are well known in the art. According to a preferred embodiment of the present invention sequence homology is determined using the BLAST Software.

The present invention is directed to a nucleic acid comprising a sequence encoding a modified glutenin polypeptide, wherein the sequence encoding one or more cysteine residues responsible for intermolecular crosslinking via disulfide bridges were deleted or substituted by sequences encoding other amino acids without amending the reading frame of the coding sequence.

Alignment of different types of LMW subunits shows the distribution of cysteine residues in the amino acid sequences (Figure 2). The distribution of the cysteine residues involved in the formation of intramolecular disulfide bonds is conserved in LMW sequences and can be used for classification of the the known LMW subunit sequences.

The nucleic acid of the present invention may for example encode a polypeptide comprising the amino acid sequence shown in SEQ ID NO: 10.

According to a further embodiment the present invention is directed to a vector comprising the above nucleic acids. The vector preferably also comprises a sequence for regulating the expression of the glutenin polypeptide coding sequence. Numerous sequences for regulating the expression of a coding sequence are known in the art. Usually the coding sequence will be flanked by a promoter and a terminator sequence. Respective sequences will allow initiation and termination of transcription of the coding sequence. Preferably, the nucleic acids and vectors of the present invention further comprise sequences regulating the translation.

The promoter can be an inducible or a constitutive promoter.

According to an alternative embodiment of the present invention, the sequences regulating the expression of the sequence encoding a modified glutenin polypeptide are sequences of microbacterial or yeast origin. This embodiment allows expression of the coding sequence in bacteria or yeast and thus mass production of the glutenin polypeptide encoded by the nucleic acids of the invention in cell culture. In accordance with this embodiment of the present invention the vector further preferably comprises a signal sequence capable of directing the secretion of the glutenin polypeptide from the host cells.

In a further aspect, the present invention is directed to a glutenin polypeptide encoded by one of the above nucleic acids. The polypeptide for example may have a sequence as shown in SEQ ID NO: 10.

According to a further embodiment the polypeptides of the present invention are used as gliadin substitutes. The polypeptides may for example be used for the preparation of foodstuffs, such as flour, dough, batters, pastries, cookies, pasta, waffers, bread and/or confectionery or for the preparation of pharmaceutical products, such as tablets. These foodstuffs will contain a considerably reduced amount of gliadin proteins or no gliadin proteins. The foodstuffs will contain the modified polypeptides of the present invention and may in addition contain unmodified LMW and/or HMW glutenin polypeptides.

In a further aspect the present invention is directed towards a host cell comprising a vector or a nucleic acid of the present invention. The vector may in addition contain genes encoding unmodified LMW and/or HMW glutenin polypeptides. The host cell can be any host cell, although bacterial cells or yeast cells are especially preferred.

A further embodiment of the present invention is directed towards a method of preparing a nucleic acid as described above, which method comprises steps wherein:
(a) a nucleic acid encoding a glutenin polypeptide is identified;
(b) the sequences within the nucleic acid of (a) are identified which encode cysteine residues responsible for intermolecular crosslinking of the glutenin polypeptide via disulfide bridges; and
(c) the nucleic acid of (a) is modified to change the sequences encoding cysteine residues identified in (b) to a sequence encoding a different amino acid without changing the reading frame of the coding sequence.

Further, methods of preparing a modified LMW glutenin polypeptide are provided, which methods comprise steps wherein:
(a) a microorganism is transfected with a nucleic acid according to the present invention; and
(b) the polypeptide is isolated from the culture supernatant or the microorganism.

In a specifically preferred embodiment of the present invention a foodstuff is provided, which does not contain any gliadin polypeptide. The foodstuff can be prepared from recombinant glutenin polypeptides expressed from yeast or bacteria transfected or transformed with the nucleic acids of the present invention and may further be supplemented with unmodified HMW-glutenin and LMW-glutenin subunits either of natural or of recombinant origin. This will allow to maintain advantagous features of respective foodstuffs prepared from wheat.

The foodstuff may advantageously be consumed by patients with coeliac disease or general intolerance to gluten.

According to an especially preferred embodiment foodstuff is prepared from recombinantly expressed polypeptides and used together with unmodified HMW-glutenin and LMW-glutenin proteins to obtain the advantagous properties of respective foodstuffs prepared from wheat for the preparation of a flour to supplement wheat and non-wheat flours. The flour can be used for the preparation of dough, pastries, cookies, pasta, bread, batters and/or confectionary.

For the above uses recombinantly expressed polypeptides can be purified for example using HPLC methods. The polypeptides can be reoxidized after HPLC (see for example Verbruggen et al., J. Cereal Science, vol. 33 (2001), 253-260). The proteins can be used in combination with other wheat proteins, that confer the visco-elastic properties, as additive for non-wheat flours, for example from rice, maize or millet, that do not cause coeliac disease and less severe forms of gluten intolerance for the preparation of several foodstuffs.

According to a preferred embodiment the foodstuff is a dough, pastry, cookie, pasta, bread, batters and/or confectionary.

The invention is also directed to the combined expression of several storage protein types in yeast or bacteria. The combination of recombinantly expressed LMW-, HMW- and modified LMW-subunits in yeasts allows production of gluten-like proteins in yeast without coeliac toxicitiy for use as additives in non-wheat flours for the preparation of several foodstuffs like dough, pastry, cookie, pasta, bread, batters and/or confectionary.

In an alternative embodiment the present invention provides methods for preparing a pharmaceutical composition comprising the preparation of a tablet using a modified LMW glutenin polypeptide according to the present invention. The modified LMW glutenin polypeptide is preferably coated onto a tablet. The present invention also relates to the tablets comprising the modified glutenin polypeptides of the present invention.

The following examples show that it is possible to alter the LMW glutenin structure in such a manner that intermolecular crosslinking by disulfide bridges is inhibited. The yeast *Saccharomyces cerevisiae* and the bacterium *Escherichia coli* were used as hosts for heterologous expression of a respective gene, as the same may perform posttranslational modifications, such as disulfide bond formation, also observed when these genes are expressed in their original hosts. Further the genome of these hosts (the yeast *Saccharomyces cerevisiae* and the bacterium *Escherichia coli*) can easily be manipulated using molecular techniques.

The expression of α-gliadin and γ-gliadin genes in yeasts has been reported (see for example Blechl et al., Gene, vol. 116 (1992), 119-127; Neill et al., Gene, vol. 55 (1987), 303-317; and Scheets and Hedgcoth, J. Agric. Foodchem., vol. 37 (1989), 829-833). However, these studies were all focused on the analysis of the structure and function of recombinantly expressed gliadins and used the yeast as a host system for that purpose.

The expression of the α-gliadin gene in yeast has also been suggested (US 4,826,765) with the purpose to use the yeast strains to supplement wheat and non-wheat flours for baked products and for use in diagnostic treatment of illness in humans caused by gluten proteins.

### Brief Description of the Figures

- Fig. 1:: Schematic diagram comparing the domain structure and showing the disulfide bridges of α-gliadin, γ-gliadin and LMW subunits of glutenin.
- Fig. 2:: Alignment of different types of LMW subunits and distribution of cysteine residues. Cysteine residues responsible for disulfide bond formation of LMW subunits are boxed. Cysteine residues at positions 237, 245, 265, 272, 273 and 385 are involved in the formation of intramolecular disulfide bonds. Highly conserved cysteine residues at position 335, conserved cysteine residues at position 25 and atypically distributed cysteine residues at positions 66 and 253 are involved in the formation of intermolecular disulfide bonds.
- Fig. 3:: Yeast expression plasmid YpADH ΔA URA3.
- Fig. 4:: Nucleotide sequence of clone LMW6 (911 base pairs) (SEQ ID NO:1).
- Fig. 5:: Amino acid sequence of clone LMW6 (298 amino acids) (SEQ ID NO:2); the cysteine residues responsible for intramolecular disulfide bond formation are marked in bold letters; the cysteine residues responsible for intermolecular disulfide bond formation are marked as bold and underlined letters.
- Fig. 6:: LMW6 subunit expression plasmid YpADH LMW6.
- Fig. 7:: SDS-PAGE and Western-blot analysis of yeast transformed with plasmid YpADH LMW6; different cell fractions were separated on SDS-PAGE.
- Fig. 8:: Schematic presentation of the PCR strategy for *in vitro* mutagenesis.

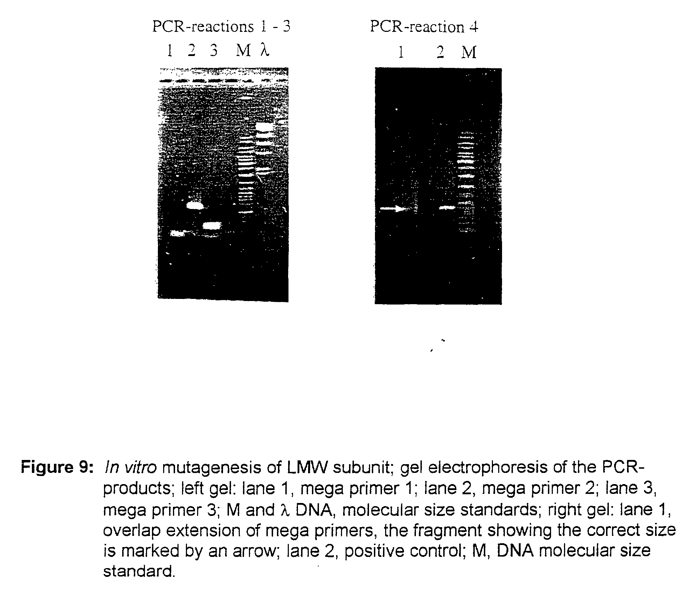

- Fig. 9:: In vitro mutagenesis of LMW subunit; gel electrophoresis of the PCR-product; left gel: lane 1, mega primer 1; lane 2, mega primer 2; lane 3, mega primer 3; M and λ DNA, molecular size standards; right gel: lane 1, overlap extension of mega primers, the fragment showing the correct size is marked by an arrow; lane 2, positive control; M, DNA molecular size standard.
- Fig. 10:: Nucleotide sequence of the modified LMW a3Δcys2gene (908 base pairs) (SEQ ID NO:9); the modified sequences are marked in bold and underlined.
- Fig. 11:: Amino acid sequence of modified LMW a3Δcys2 protein (297 amino acids) (SEQ ID NO:10); the modified parts are underlined.
- Fig. 12:: Western blot analysis of E. coli transformands; lanes 1 and 2: strain BL21(DE3) pET28aLMW6 not induced (1) and induced with 100 mM IPTG (2); lanes 3 and 4: strain BL21(DE3) pET28aLMWa3Δcys2 not induced (3) and induced with 100 mM IPTG (4); lanes 5 and 6: strain BL21 (DE3) LysS pET28aLMW6 not induced (5) and induced with 100 mM IPTG (6); lanes 7 and 8: strain BL21(DE3)LysS pET28aLMWa3Δcys2 not induced (7) and induced (8) with 100 mM IPTG (8); M: protein standard.
- Fig. 13:: Western blot analysis of yeast transformands; lanes 1 and 6: AH22ura3:YpADHΔURA3, empty control plasmid; lanes 2 and 7: AH22ura3:YpADH LMW6, wild type LMW-glutenin; lanes 3 and 8: AH22ura3:YpADH γ-gliadin, monomeric reference; lanes 4 and 9: AH22ura3:YpADH LMWa3Δcys, modified LMW subunit; lane 5: protein standard.
- Fig. 14:: Extraction of wheat storage proteins from yeast cells. SDS-PAGE: lane 1: crude cell extract; lane 2: pre extract; lane 3: main extract; M: molecular weight standard; lane 4: purified LMW subunit.
- Fig. 15:: HMW Dx5 subunit expression plasmid YpADH HMW Dx5.
- Fig. 16:: HMW Dy10 subunit expression plasmid YpADH HMW Dy10.
- Fig. 17:: SDS-PAGE of isopropanol extracts (main extracts) from yeast transformands producing HMW subunits; lane 1: HMW Dx5 subunit; lane 2: HMW Dy10 subunit; M: molecular weight standard.

### Materials and Methods

The following materials and methods were used in the subsequent examples:
Strains
*Escherichia coli* SF8: recBC, recC22, lop11, tonAl, thr1, leuB6, thy1, lacY1, supE44, hsdM⁻ , hsdrR⁻, Sm^{r}, λ⁻ (Struhl et al., Procl. Natl. Acad. Sci. USA, vol. 73 (1976), 1471 - 1475).
BL21 (DE3): F-ompT hsdS B (r B-m B-) gal dcm (DE3) (Novagen)
BL21 (DE3)LysE: F-ompT hsdS B (r B-m B-) gal dcm (DE3)pLysS (CmR) (Novagen)
*Saccharomyces cerevisiae* AH22ura3: MATa; leu2-3; leu2-112; his4-519; canl; ura3Δ: cir⁺ (Polakowski T. (1999) Molekularbiologische Beeinflussung des Ergosterolstoffwechsels der Hefe *Saccharomyces cerevisiae*. Dissertation, Technische Universität Berlin, Shaker Verlag, Aachen).

Plasmids
YpADH-AΔURA3: E.coli/S.cerivisiae shuttle vector carrying the yeast specific ADH1 promoter and URA3 gene as selectable marker.
YpADH-γE1/C2: vector YpADH-AΔ-URA3 carrying γ-glutenin subunit from plasmid pW1020 (Sheets and Hedgcoth, Plant Sci., vol. 57 (1988), 141-150).
PET-28a(+): (Novagen)

Oligonucleotides
The following oligonucleotides were used for cloning or for in vitro mutagenesis:

LMW-E1: 5'-CCG-GAA-TTC-ATC-ATG-AAG-ACC-TTC-CTC-3' (SEQ ID NO:3)

LMW-C2: 5'-CCA-TCG-ATC-AGT-AGG-CAC-CAA-CTC-GG-3' (SEQ ID NO:4)

LMW-Af: 5'-AGA-CTA-GCA-TCC-CTG-GTT-TGG-3' (SEQ ID NO:5)

LMW-Ar: 5'-CCA-GGG-ATG-CTA-GTC-TCC-ATC-3' (SEQ ID NO:6)

LMW-Bf: 5'-GCT-CGG-ACA-AGG-TTC-TTT-CC-3' (SEQ ID NO:7)

LMW-Br: 5'-GGA-AAG-AAC-CTT-GTC-CGA-GC-3' (SEQ ID NO:8)

Media and growth conditions:

| | |
|---|---|
| LB medium | 10 g peptone, 5 g yeast extract, 10g NaCl per liter, adjusted to pH7.5 with NaOH, |
| YEPD medium | 20 g glucose, 20 g peptone, 10 g yeast extract per liter. |
| YNB medium | 6,7 g yeast nitrogen base (YNB, Difco), 20 g glucose, 100 mg leucine per liter. |
| DS medium | 50 g sucrose, 5,0 g (NH₄)₂SO₄, 10,0 g Na-glutamate, 3, 2 g (NH₄) H₂PO₄, 1,5 g KCl, 0,75 g MgSO₄, 0,1 g CaCl₂, 0,1 g myo-inositol, 2,5 g casamino acids (Difco), 0,3 g leucine per liter. |

Vitamin solution: 11,5 mg biotin, 2,0 g D-pantothenic acid, 2,0 g pyridoxine, 2,0 g thiamine, 2,0 g nicotinic acid per liter.

Trace element solution: 1,0 g FeSO₄x7H₂O, 3,5 g ZnSO₄x7H₂O, 0,2 g CuSO₄x5H₂O, 0,2 g NaMoO₄x2H₂O, 3,5 g MnSO per liter.

4 ml/l of vitamin solution and 2 ml/l of trace element solutions were added to the medium after autoclaving and cooling.

E. coli strains were cultivated in LB medium at 37°C. Transformands were cultivated in LB with the addition of 100 µg/ml ampicilline or 30 µg/ml kanamycin for selection. Solid media was obtained by adding 1.5 % agar agar.

S. cerevisiae were cultivated either in complex medium YEPD or in synthetic medium YNB or DS at 28°C. Solid media was obtained by adding 1.5 % agar agar.

10 liter lab-scale yeast fermentation:

| Batch medium | |
|---|---|
| DS medium | 50 g sucrose, 5,0 g (NH₄)₂SO₄, 10,0 g Na-L-glutamate, 3, 2 g (NH₄) H₂PO₄, 1, 5 g KCl, 0, 75 g MgSO₄x7H₂O, 0,1 g CaCl₂x2H₂O, 0,1 g myo-inositol, 4 ml vitamin solution, 1ml FeSO₄ per liter. |
| | |
| Feeding medium | 100 g sucrose, 7,0 g (NH₄)₂SO₄, 20,0 g Na-L-glutamate, 1,9 g (NH₄)H₂PO₄, 1,0 g MgSO₄, 0,2 g CaCl₂, 0,2 g myo-inositol, 2,0 g L-leucine, 10 g casamino acids (Difco), 8 ml vitamin solution, 2 ml trace element solution, 2ml FeSO₄ per liter. |
| | |
| Vitamin solution | 11,56 mg biotin, 2,036 g D-pantothenic acid calcium salt, 2,0 g pyridoxine, 2,0 g thiamine, 2,0 g nicotinic acid per liter. Stored at 4°C. |
| | |
| Trace element solution | 0,349 g ZnSO₄x7H₂O, 0,02 g CuSO₄x5H₂O, 0,02 g NaMoO₄x2H₂O, 0,349 g MnSO₄xH₂O per 50 ml. Stored at 4°C. |
| | |
| FeSO₄ solution | 2,0 g FeSO₄x7H₂O per liter. Stored at -20°C. |

### Recombinant DNA Techniques

Recombinant DNA techniques with *Escherichia coli* were carried out as described in the pET System Manual (TB055, 9^{th} edition, 05/2000) by Novagen.

If not otherwise mentioned, standard recombinant DNA techniques described by Sambrook and coworkers (Molecular Cloning, 1989) were used.

### Example 1

### Cloning of LMW subunit genes from wheat cultivar Cheyenne

The wheat genome contains more than 20 different genes coding for LMW subunits of glutenin. The aim of this work was to isolate a LMW subunit not containing the epitope QQQPP, which has been reported to cause allergenic reactions.

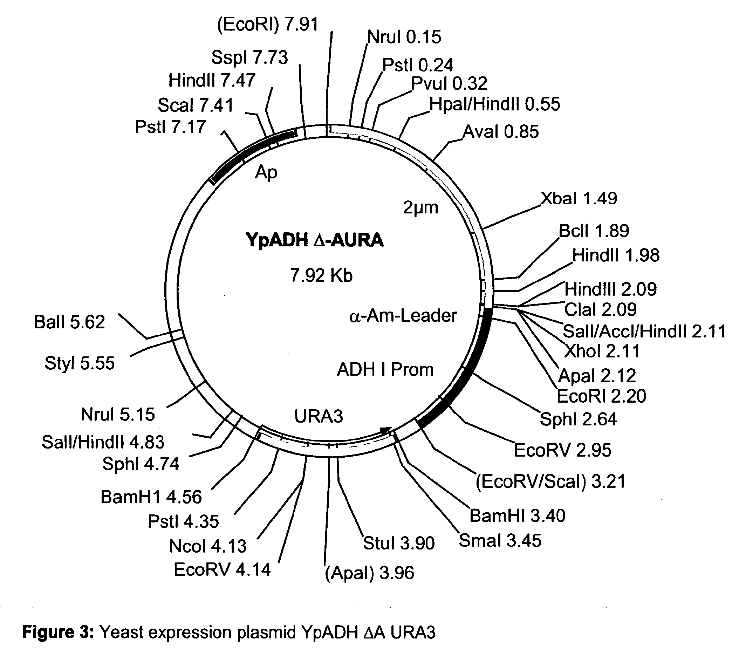

LMW subunit genes were isolated from wheat genomic DNA of the cultivar Cheyenne by polymerase chain reaction (PCR). Genomic DNA from the cultivar Cheyenne was provided by Dirk Becker, University of Hamburg. Since all LMW subunit genes known so far have homologous 5'- and 3'-ends, primers amplifying the entire LMW subunit gene including the region coding for the signal peptide, were designed according to the published sequences. In order to clone the amplified PCR fragments directly into the yeast expression vector YpADH-DA URA3 (Fig. 3), the forward primer LMW-E1 (SEQ ID NO:3) includes a novel *Eco*RI site and the reverse primer LMW-C2 (SEQ ID NO:4) a novel *Cla*l site. PCR-amplification was done in 20 µl of the following reaction mixture: 9.8 µl H₂O, 2 µl 10 x dNTP'S (MBI-Fermentas), 5 µl 4 x reaction buffer (MBI-Fermentas), each 1 µl 10 pmol primer, 1 µl template DNA (100 ng of genomic DNA), 0.2 µl Taq polymerase (4 U/µl, MBI-Fermentas). PCR conditions were: initial step at 94°C for 2 min, followed by 30 cycles at 94°C for 15 sec, 60°C for 20 sec, 72°C for 35 sec, and a final step at 72°C for 2 min.

PCR products were analysed by electrophoresis using 1.2 % agarose gels and subsequently purified using the QIAquick PCR purification kit (Qiagen). The purified PCR fragments were digested with *Eco*RI and *Cla*l and ligated into the yeast expression vector YpADH-ΔA URA3. E.coli strain SF8 was transformed with the ligation mix. E.coli clones were analysed by PCR for carrying LMW subunit inserts followed by restriction analysis. The DNA-inserts of clones showing different restriction patterns were further characterized by sequencing.

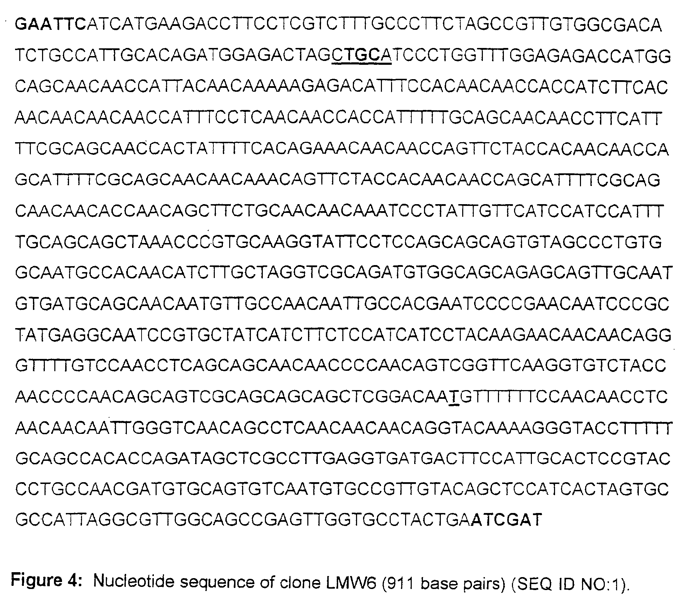

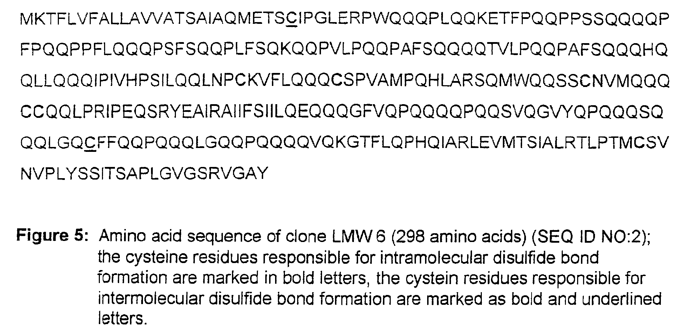

Several clones were obtained distinguishable by different restriction patterns, which were further characterized by sequencing. The resulting nucleotide sequences were compared with published sequences for LMW subunit genes by computer analysis. Sequence of clone LMW6 (Figs. 4 and 5, SEQ ID NO:1 and 2) does not contain the allergenic epitope QQQPP and shows some, although minor, differences to published sequences, therefore representing a new allel for a LMW subunit gene not published before.

### Example 2

### Modification of LMW subunit gene of clone LMW 6

In order to change the aggregative nature (behavior) of LMW subunits the 2 cysteine residues responsible for intermolecular disulfide-bond formation cysteine 1 in domain I and cysteine 7 in domain IV (marked in Fig. 3 as bold and underlined letters) have to be removed, either by deletion or modification of the coding triplet.

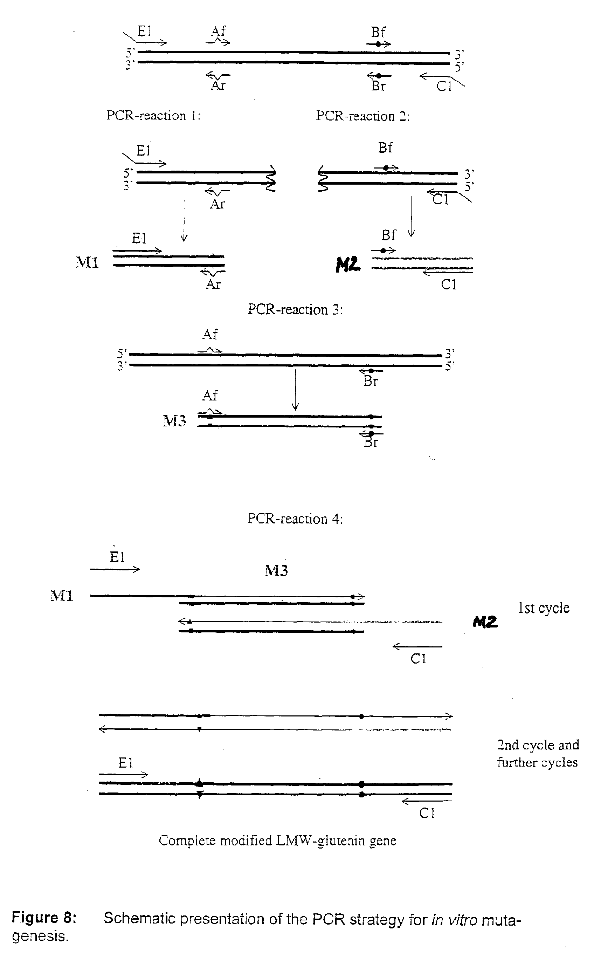

Site directed modification of the LMW subunit gene was performed by in vitro mutagenesis using a PCR-strategy. The principle of the method is shown in Figure 8. Two overlapping primer pairs, one for each modification, carrying the desired mutations, were created. Three mega primers with the desired mutations were amplified in the first step. The reaction mixtures contained: 22.8 µl H₂O, 4 µl 10 x dNTP's (MBI-Fermentas), 10 µl 4 x reaction buffer (MBI-Fermentas), 1 µl template DNA (100ng of YpADH LMW6), 0.2 µl Taq polymerase (4 U/µl, MBI-Fermentas), and each 1 µl 10 pmol of the following primer pairs:

PCR1: primer LMW-E1 (SEQ ID NO:3) and LMW-Ar (SEQ ID NO:6)

PCR2: primer LMW-Af (SEQ ID NO:5) and LMW-Br (SEQ ID NO:8)

PCR3: primer LMW-Bf (SEQ ID NO:7) and LMW-C2 (SEQ ID NO:4)

PCR conditions were the same as for amplifying LMW subunit genes, but without the last elongation step for 2 min to prevent A-tailing of the PCR-products.

The resulting PCR-products were analysed by gel electrophoresis and purified using the QIAquick PCR purification kit (Qiagen).

The 3 mega primers were joined together in the second step by overlap extension in order to amplify a complete gene, 4 µl of each mega primer, 2 µl of each primer LMW-E1 and LMW-C2, 4 µl 10 x dNTP'S (MBI-Fermentas), 10 µl 4 x buffer (MBI-Fermentas) and 2.0 µl Taq polymerase (4 U/µl, MBI-Fermentas) were mixed in one tube. PCR conditions were the same as above, except using a longer elongation time for 1 min. The resulting PCR-products were analysed by gel electrophoresis, purified, restricted with *Eco*RI and *Cla*l and cloned in vector YpADG-ΔA URA3 as described above. Transformands were analysed by PCR and restriction analysis and the modification of the LMW subunit gene was confirmed by sequencing.

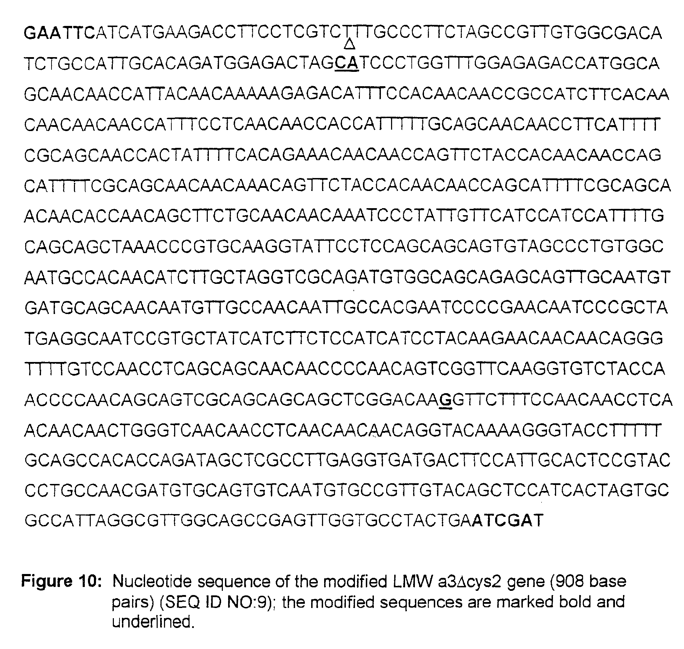

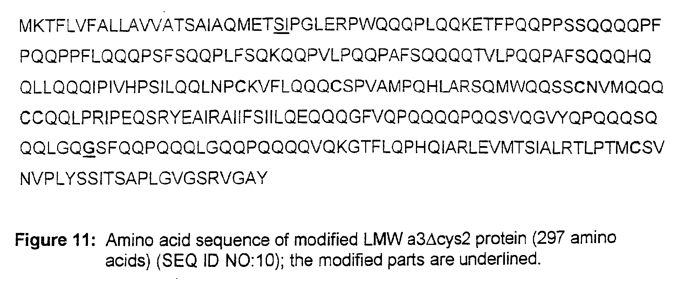

The resulting modified LMW subunit gene and the yeast expression vector were named LMWa3Δcys2 and YpADH LMWa3Δcys2, respectively. DNA and amino acid sequences of modified LMWa3Δcys2 are shown in Figures 10 and 11 (SEQ ID NO:9 and 10).

### Example 3

### Expression of modified glutenin genes in Eschericia coli and analysis of bacterial proteins

For the expression of LMW subunits in E.coli the Novagen (Novagen Inc., 441 Charmany Drive, Madison, WI 53719, USA) pET system, vectors and strains were used.

In order to clone LMW6 (SEQ ID NO:1) and modified LMWa3Δcys2 (SEQ ID NO:9) gene into the E.coli expression vector pET28 (a) the yeast expression plasmids YpADH LMW6 and YpADHLMWa3Δcys2 were digested with the restriction enzymes BspH1 and HindIII to release the LMW coding sequences. The purified LMW fragments were ligated into the *Nco*I and *Hind*III digested pET28(a) vector. The ligation mix was transformed into E.coli strain SF8 and transformands were selected on LB agar plates containing kanamycin. E.coli clones were analysed by PCR for carrying LMW subunit inserts followed by restriction analysis. The plasmids were named pET28a LMW6 and pET28a LMWa3Δcys2.

For protein expression strains BL21 (DE3) und BL21(DE3)LysE were transformed with the plasmids pET28a LMW6 and pET28a LMWa3Δcys2. Induction of gene expression was carried out as described in the manual of Novagen.

Protein epxression was performed a described in the manual. 1 ml samples were taken from the cultures and the cells harvested by centrifugation. The cell pellet was suspended in 100 µl sample buffer (with DTT) and boiled for 5 minutes. 10 µl of each sample was loaded on a 15 % SDS-polyacrylamid gel and the separated proteins were transferred to nitrocellulose membrane afterwards. Immunodetection was carried out using a mouse anti-gliadin antibody specific to both gliadin and LMW-glutenin subunits. Immunoreacting bands were detected using POD conjugated anti-mouse antibody and H₂O₂ as detection substances.

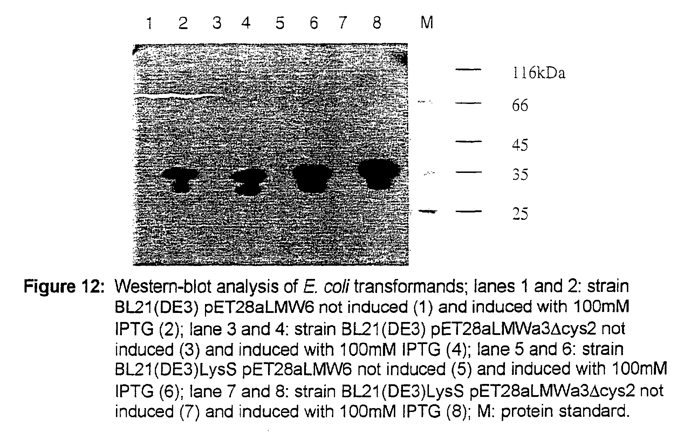

As shown in Figure 12 the native LMW6 subunit as well as the modified LMWa3Δcys2 subunit were produced in E.coli after induction with IPTG and no protein was produced by an uninduced culture. The expression level of strain BL21 (DE3) LysE is slightly than that of of strain BL21 (DE3). The LMW subunits produced in E.coli have a slightly larger size than the native protein, because E. coli cells will not perform the processing of the N-terminal leader sequence. This results show that E.coli could also be used as an alternative expression system in order to produce large amounts of the modified LMW subunit.

### Example 4

### Transformation of Saccharomyces cerevisiae and isolation and analysis of yeast proteins

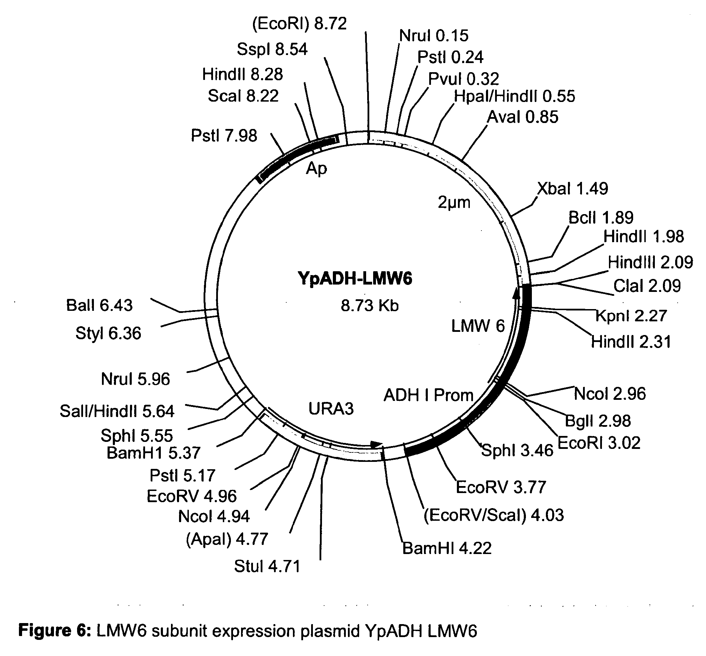

Yeast strain AH22ura3 was transformed using a modified electro-poration protocol described by Becker and Guarente (Methods Enzymol., vol. 194 (1991), 182-187) with vectors YpΔAH LMW6 (Fig. 6), YpAΔH-LMWa3Δcys2, YpADH-γE1/C2 and the empty control vector YpADH-AΔURA3 and protein production was investigated by SDS-PAGE analysis and Western-blot detection. Transformands were selected on YNB agar lacking uracil.

Yeast total cellular proteins were prepared from transformed yeast in DS medium lacking uracil. Cells were collected by centrifugation and disrupted with glass beads. Cell debris was suspended in sample buffer (with or without dithiothreitol DTT) heated for 3 min in a boiling water bath prior to loading on the gel.

In order to analyse the polymeric or monomeric nature of the proteins SDS-PAGE analysis was carried out under both reducing and non-reducing conditions. The total cellular proteins (reduced and non-reduced samples) were separated on a 15 % SDS-polyacrylamid gel and transferred to nitrocellulose membrane. Immunodetection was carried out using a mouse anti-gliadin antibody specific to both gliadin and LMW-glutenin subunits. Immunoreacting bands were detected using POD conjugated anti-mouse antibody (Roche) using Luminol and H₂O₂ as detection substances.

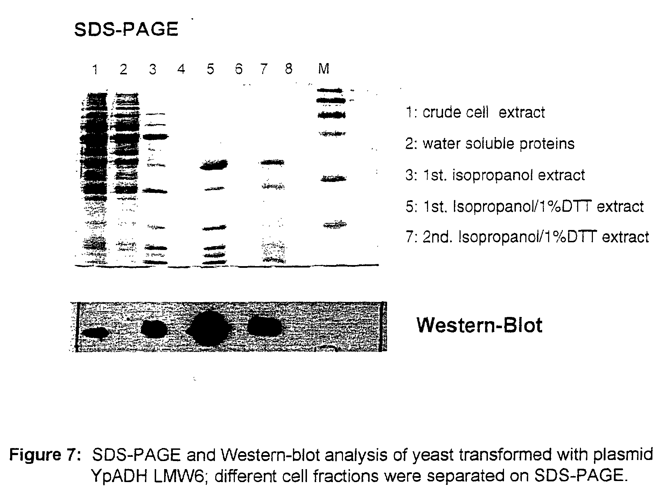

As shown in Figure 7, a single protein band of approx. 35 kDa, consistent with the predicted size for mature LMW6 subunit could be detected for yeast cell extracts. The major part of the LMW protein could be extracted from disrupted cells using 50 % isopropanol containing 0.5 % DTT. This is the first report on the expression of LMW subunit genes and the production of a mature LMW subunit protein in the yeast *Saccharomyces cerevisiae.*

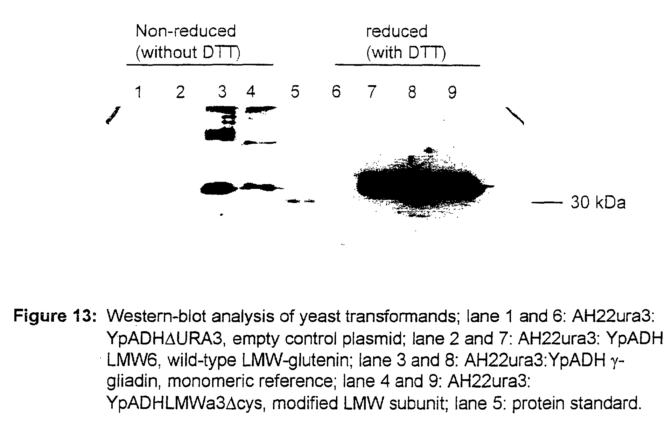

The heterologous proteins produced in yeast were detected by Western-blot analysis using an antibody specific to gliadin and LMW-glutenin subunits. The Western-blot is shown in Fig. 13. The monomeric forms of all proteins, the wild type LMW subunit, the modified LMW subunit and the γ-gliadin, could be detected under reducing conditions as single bands at a size of approx. 35 kDa.

Under non-reducing conditions, only the monomeric forms of γ-gliadin and the modified LMW subunit could be detected, where a monomeric wild-type LMW subunit could not be detected. The genetically modified LMW subunit has an altered protein structure and behaves in a gliadin like monomeric manner. It was shown for the first time in vivo that cysteine 1 and cysteine 7 of LMW subunit are involved in intermolecular disulfide-bond formation and that the deletion of these cysteine residues results in a monomeric protein structure.

The modified LMW subunit produced in yeast cells can therefore be used as a functional substitute for gliadin as a "solvent" for aggregated glutenins, for example, in gluten-free foods for patients with coeliac disease. Furthermore, the results indicate that disulfide bond formation of wheat endosperm proteins produced in yeast is similar to the formation of disulfide bonds in wheat plants: the wild type LMW subunit forms polymeric structures, whereas the modified LMW subunit is present in a monomeric form similar to γ-gliadin. The yeast S. cerevisiae is therefore a suitable host organism for the heterologous production of gluten proteins.

### Example 5

### Production and purification of wheat storage proteins from yeast cells

Fermentation of yeast transfected as outlined above was performed in fed batch mode.

Fed batch processes were performed at 27 - 30°C and pH5. Dissolved oxygen was measured with a galvanic (Pt/Pb) electrode. During the fermentation the bioreactor was aerated with an air flow of 1 vvm (volume air/volume and minute) and agitated between 600 and 1000 rpm. Dissolved oxygen content was between 90% saturation at the beginning and 20 % at the end of the fermentation. The pH was maintained at 5.0 by addition of 4 M KOH. Foam, if necessary, was suppressed by the addition of polypropylene glycol 2000.

The feeding rate was controlled by a computer system (Fed-Batch Control 2.01, P. Götz/L. Baumann, Berlin 2002) regulating the feeding rate of the growth medium in order to get a constant growth rate µ of 0.14 per hour.

### Cell disruption

At the end of the fermentation yeast cells were harvested by centrifugation and the cells were suspended in PBS disruption buffer ((PBS: 10mM phosphate pH 7.4, 150 mM NaCl), 1 mM Phenylmethylsulfonylfluorid (PMSF), 0.5 % Triton X-100) in a ratio of 0.5 ml disruption buffer per gram cell-wet weight. Cells were disrupted within a DYNO-MILL (Laboratory mill type KDL, Willy A. Bachofen, Basel, Switzerland) using glass beads with a diameter of 0.75 - 1.00 mm.

### Extraction of wheat storage proteins from yeast cells

The disrupted yeast cell suspension (crude cell-lysate) was mixed with an equal volume of isopropanol and centrifuged. The supernatant (pre extract) was discarded and the pellet was reextracted 1 to 2 times with 50 % isopropanol and 1 % DTT at 60°C for 2 hours. Additionally an ultrasound sonication was performed for 10 minutes. The suspension was cooled down to room temperature and centrifuged. The supernatant (main extract) was collected and the proteins were precipitated by increasing the isopropanol concentration to 83 %. The precipitate was collected by centrifugation, washed two times with PBS buffer and freeze dried.

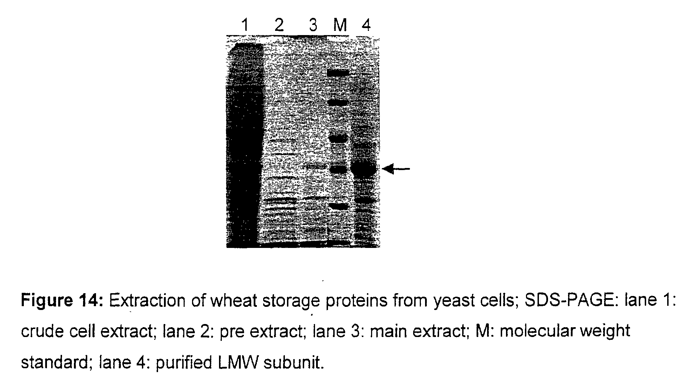

Wheat storage proteins from yeast cells in the several fractions of the extraction after SDS-PAGE are shown in Fig. 14.

Further analysis of heterologous expressed proteins in yeast can be carried out as described in Kipp et al. (J. Cereal Science, vol. 23 (1996), 227-234) and Kiefer et al. (J. Cereal Science, vol. 27 (1998), 53-60) except that purified proteins from yeast were mixed with rice and maize flours in order to investigate the rheological and breadmaking properties.

### Example 6

### Expression of HMW subunits in yeast

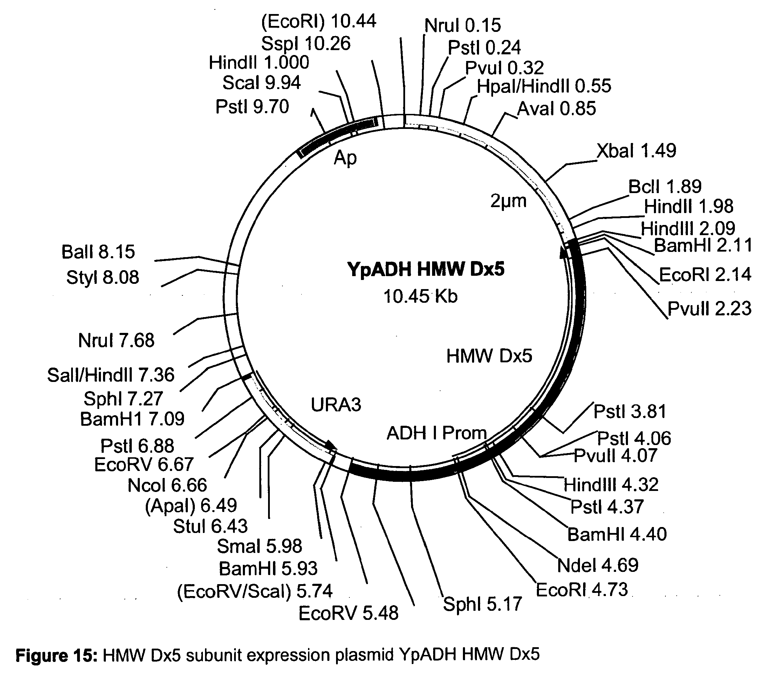

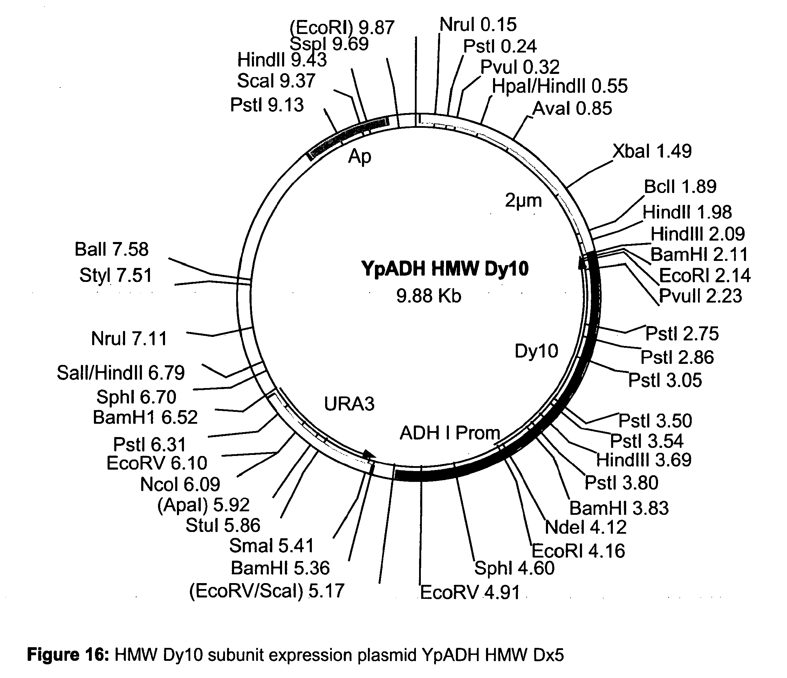

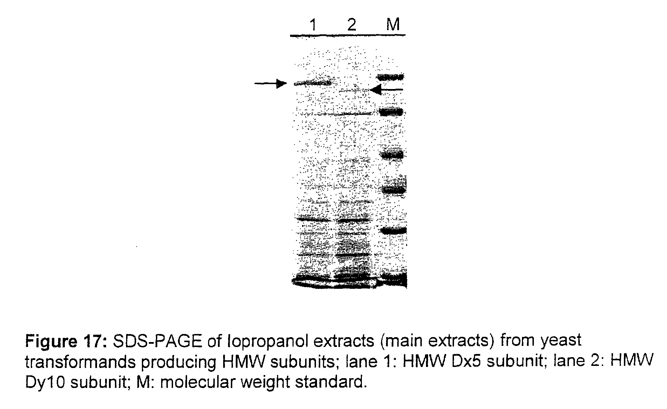

To investigate the combined expression of several of the storage proteins, Yeast cells were transformed with the HMW subunits Dx5 and Dx10 of wheat using the expression plasmids YpADH HMW Dx5 (Figure 15) and YpADH HMW Dy10 (Figure 16), respectively, and standard protocols. Transformed yeasts were grown in culture, disrupted and wheat storage proteins were extracted essentially as outlined above. Main extracts achieved by isopropanol extraction (see example 5) from the yeast transformants were analysed using SDS-PAGE. Protein bands marked with an arrow in Figur 17 represent production of the wheat HMW Dx5 and HMW Dy10 subunits in yeast. This is the first time, that expression of wheat HMW subunits was detectable in transformed yeast cells. Combined expression in yeast of HMW-, modified and unmodified LMW subunits will provide protein mixtures with gluten-like structures in yeast. After purification these protein structures will not cause coeliac toxicity and can be used as supplement for non-wheat flour to maintain the advantagous features of respective foodstuffs prepared from wheat flour.

## Claims

1. Nucleic acid comprising a sequence encoding a modified glutenin polypeptide, wherein the sequence encoding one or more cysteine residues responsible for intermolecular crosslinking via disulfide bridges were deleted or substituted by sequences encoding other amino acids without amending the reading frame of the coding sequence.

2. Nucleic acid according to claim 1, which is a DNA or an RNA.

3. Nucleic acid according to one of claims 1 or 2, wherein the glutenin coding sequence is a sequence having a homology of at least 75 % to the sequence shown in SEQ ID NO: 9.

4. Nucleic acid according to any of the preceding claims, wherein the nucleic acid encodes a polypeptide in which the cysteine residue in domain I and/or the cysteine residue in domain IV have been deleted or substituted by another amino acid.

5. Nucleic acid according to any of the preceding claims, wherein the modified glutenin polypeptide is maintained as a monomer with a size of less than 200 kDa, preferably less than 100 kDa at conditions which will allow disulfide bond formation.

6. Nucleic acid according to any of claims 1 to 5, wherein the nucleic acid encodes a polypeptide comprising the amino acid sequence shown in SEQ ID NO: 10.

7. Nucleic acid according to any of the preceding claims, wherein the nucleic acid further encodes unmodified LMW and/or unmodified HMW glutenin polypeptides.

8. Vector comprising a nucleic acid according to any of claims 1 to 7.

9. Vector according to claim 8 which further comprises a sequence for regulating the expression of the glutenin polypeptide.

10. Vector according to claim 8 or 9, wherein the sequence regulating the expression of the glutenin polypeptide comprises a promoter and a terminator sequence.

11. Vector according to any of claims 8 to 10, wherein the promoter is an inducible promoter.

12. Vector according to any of claims 8 to 10, wherein the promoter is a constitutive promoter.

13. Vector according to any of claims 8 to 12, wherein the promoter is a microbacterial or yeast promoter.

14. Vector according to any of claims 8 to 13, wherein the vector further comprises a signal sequence capable of directing the secretion of the glutenin protein.

15. Polypeptide encoded by a nucleic acid according to anyone of claims 1 to 14.

16. Polypeptide according to claim 15, comprising the sequence shown in SEQ ID NO: 10.

17. Use of a polypeptide according to claim 15 or 16 as a gliadin substitute.

18. Use according to claim 17, wherein the use comprises the combined use of a modified glutenin polypeptide, wherein the sequence encoding one or more cysteine residues responsible for intermolecular crosslinking via disulfide bridges were deleted or substituted and unmodified LMW and/or unmodified HMW glutenin polypeptides.

19. Use according to claim 17 or 18, wherein the polypeptide is used for the preparation of foodstuffs, such as flour, dough, batters, pastries, cookies, pasta, waffers, bread and/or confectionery or for the preparation of pharmaceutical products, such as tablets.

20. Use according to any of claims 18 or 19, wherein the foodstuffs contain a considerably reduced amount of gliadin proteins or no gliadin proteins.

21. Host cell comprising a vector according to one of claims 8 to 14.

22. Host cell according to claim 21, wherein the cell is a bacterial cell or a yeast cell.

23. Host cell according to claim 22, wherein the cell is the yeast Saccharomyces cerevisiae, the yeast Pichia pastoris or the bacterium Escherichia coli.

24. Method of preparing a nucleic acid comprising steps wherein:
(a) a nucleic acid encoding a glutenin polypeptide is identified;
(b) the sequences within the nucleic acid of (a) are identified which encode cysteine residues responsible for intermolecular crosslinking of the glutenin polypeptide via disulfide bridges; and
(c) the nucleic acid of (a) is modified to change the sequences encoding cysteine residues identified in (b) to a sequence encoding a different amino acid without changing the reading frame of the coding sequence.

25. Method of preparing a modified LMW glutenin polypeptide comprising steps wherein:
(a) a microorganism is transfected with a nucleic acid according to one of claims 1 to 14 or prepared according to 24; and
(b) the polypeptide is isolated from the culture supernatant or the microorganism.

26. Method of preparing footstuff comprising the preparation of a flour using a modified LMW glutenin polypeptide according to any of claims 15 to 17 or prepared according to claim 25.

27. Method of preparing foodstuff from recombinantly expressed polypeptides according to claim 26, wherein the modified LMW glutenin polypeptides are used for the preparation of a flour to supplement wheat and non-wheat flours.

28. Method of preparing foodstuff from recombinantly expressed polypeptides according to one of claims 26 or 27, wherein the flour is used for the preparation of dough, batters, pastries, cookies, pasta, waffers, bread and/or confectionery.

29. Foodstuff, comprising a polypeptide according to one of claims 15 to 17 or obtainable by a method according to one of claims 26 to 28, wherein the foodstuff preferably is dough, batters, pastries, cookies, pasta, waffers, bread and/or confectionery.

30. Foodstuff according to claim 29, which comprises less than 0.01 % by weight gliadin proteins.

31. Method of preparing a pharmaceutical composition comprising the preparation of a tablet using a modified LMW glutenin polypeptide according to one of claims 15 to 17 or prepared according to claim 25.

32. Method according to claim 31, wherein the modified LMW glutenin polypeptide is coated onto a tablet.

33. Tablet comprising a polypeptide according to any of claims 15 to 17 or obtainable in a method according to claim 31 or 32.
